# EUROPÄISCHE PATENTSCHRIFT

(11) **EP 0 975 622 B1**
(45) Veröffentlichungstag und Bekanntmachung des Hinweises auf die Patenterteilung: **09.10.2002**
(21) Anmeldenummer: 98922644.4
(22) Anmeldetag: 04.04.1998
(51) Int. Cl.: C07D 321/00, C07D 313/00, C07D 315/00, C07D 267/00, C07D 307/28, C07D 207/20, C07C 45/67, C07B 37/10, C08G 61/08

(54) **SELEKTIVE OLEFINMETATHESE VON BI- ODER POLYFUNKTIONELLEN SUBSTRATEN IN KOMPRIMIERTEM KOHLENDIOXID ALS REAKTIONSMEDIUM**
SELECTIVE OLEFIN METATHESIS OF BIFUNCTIONAL OR POLYFUNCTIONAL SUBSTRATES IN COMPRESSED CARBON DIOXIDE AS REACTION MEDIUM
METATHESE OLEFINIQUE SELECTIVE DE SUBSTRATS BIFONCTIONNELS OU POLYFONCTIONNELS DANS DU DIOXYDE DE CARBONE COMPRIME EN TANT QUE MILIEU DE REACTION

(30) Priorität: 18.04.1997 DE 19716231; 16.05.1997 DE 19720798
(43) Veröffentlichungstag der Anmeldung: 02.02.2000
(73) Patentinhaber: Studiengesellschaft Kohle mbH, 45470 Mülheim (DE)
(72) Erfinder: FÜRSTNER, Alois, D-45470 Mülheim (DE); LEITNER, Walter, D-45470 Mülheim (DE); KOCH, Daniel, D-45470 Mülheim (DE); LANGEMANN, Klaus, D-45470 Mülheim (DE); SIX, Christian, D-45470 Mülheim (DE)
(74) Vertreter: von Kreisler, Alek, Dipl.-Chem.
(86) Internationale Anmeldenummer: EP9801977
(87) Internationale Veröffentlichungsnummer: WO98047891

(56) Entgegenhaltungen:
- WO-A-91/14665
- WO-A-96/32421
- V.DRAGUTAN ET AL.: "OLEFIN METATHESIS AND RING OPENING POLYMERISATION OF CYCLO-OLEFINS." 1985 , J.WILEY , NEW YORK XP002077145 siehe Seite 68, Absatz 2.2.4.1. - Seite 75
- P.BERTINATO ET AL.: "STUDIES TOWARD A SYNTHESIS OF EPOTHILONE A:" JOURNAL OF ORGANIC CHEMISTRY., Bd. 61, 1996, Seiten 8000-8001, XP002077143 EASTON US in der Anmeldung erwähnt
- A.FÜRSTNER ET AL.: "CONFORMATIONALLY UNBIASED MACROCYCLISATION REACTIONS BY RING CLOSING METATHESIS." JOURNAL OF ORGANIC CHEMISTRY., Bd. 61, 1996, Seiten 3942-3943, XP002077144 EASTON US in der Anmeldung erwähnt
- K.C. NICOLAOU ET AL.: "AN APPROCH TO EPOTHILONES BASED ON OLEFIN METATHESIS." ANGEWANDTE CHEMIE. INTERNATIONAL EDITION., Bd. 35, Nr. 20, 1996, Seiten 2399-2401, XP002064440 WEINHEIM DE in der Anmeldung erwähnt

## Beschreibung

Die vorliegende Erfindung betrifft die Herstellung cyclischer Produkte durch selektive Olefinmetathese von bi- oder polyfunktionellen Substraten in Gegenwart von einem oder mehreren homogen oder heterogen vorliegenden Metathesekatalysatoren in einem Reaktionsmedium, dadurch gekennzeichnet, daß die Substrate zwei oder mehrere funktionelle Gruppen in Form von substituierten oder unsubstituierten Alken- oder Alkin-Einheiten enthalten und das Reaktionsmedium im wesentlichen aus komprimiertem Kohlendioxid besteht. Ferner betrifft die Erfindung die Herstellung cyclischer oder polymerer Produkte nach dem genannten Verfahren, wobei die Reaktionstemperatur und der Gesamtdruck so aufeinander abgestimmt sind, daß die Dichte des Reaktionsmediums im Bereich von *d* = 0.2 - 1.5 g cm⁻³ liegt und die Produktverteilung im wesentlichen durch die Dichte des Reaktionsmediums gesteuert wird.

Unter Olefinmetathese versteht man die wechselseitige Umalkylidenierung von Alkenen. Reaktionen dieser Art werden in der Regel durch Metallverbindungen katalysiert (Übersicht: Ivin, K. J.; Mol, J. C. *Olefin Metathesis and Metathesis Polymerization*, Academic Press, New York, **1997**) und finden Anwendungen in einer Vielzahl technisch bedeutender Prozesse. Dazu zählt unter anderem die Darstellung von Alkenen, beispielsweise im Shell-Higher-Olefin-Prozeß (Sherwood, M. *Chem. Ind. (London)* **1982,** 994), im Phillips-Triolefin-Prozeß und in der Produktion von α,ω-Diolefinen (Banks, R. L. et al. *J. Mol. Catal*. **1982,** *15*, 21). Eine weitere Anwendung stellt die ringöffnende Oligomerisation bzw. Polymerisation von Cycloalkenen (ROMP, **US Patent** 4,567,244) dar, die z. B. zur Produktion von Vestenamer® (Dräxler, A. *Der Lichtbogen* **1986,** *35,* 24) oder Norsorex® (Ohm, R. F. *Chemtech* **1980,** 198) genutzt wird. Weiters sind die Oligomerisation bzw. Polymerisation von acyclischen Dienen (ADMET, Lindmark-Hamberg, M. et al. *Macromolecules* **1987,** *20,* 2951), die Synthese von Carbo- und Heterocyclen unterschiedlicher Ringgrößen durch Ringschlußmetathese (RCM, **WO** 96/04289, Grubbs, R. H. et al. *Acc. Chem. Res*. **1995,** *28,* 446), gekreuzte Metathesen unterschiedlicher Alkene (Brümmer, O. et al. *Chem. Eur. J.* **1997,** *3,* 441), sowie Enin-Metathesen (Kinoshita, A. et al. *Synlett* **1994,** 1020; Kim, S.-H. et al. *J. Org. Chem*. **1996,** *61,* 1073) zu nennen. Diverse Carbo- oder Heterocyclen mit Ringgrößen von ≥ 5, die mit Hilfe der RCM dargestellt worden sind, wurden bereits zur Synthese von natürlichen oder synthetischen Wirkstoffen, Geruchs- und Geschmacksstoffen, Pheromonen, Pharmaka, Kronenethern etc. genutzt (US Patentanmeldung 08/767.561 vom 16.12.1996, Studiengesellschaft Kohle mbH). Besondere Erwähnung finden soll die effiziente Synthese von Makrocyclen durch RCM (Fürstner, A. et al. *J. Org. Chem*. **1996,** *61,* 3942), die u. a. die Grundlage mehrerer Synthesen des Antitumorwirkstoffs Epothilon und seiner Analoga bildet (Bertinato, P. et al. *J. Org. Chem*. **1996,** *61,* 8000; Nicolaou, K. C. et al. *Angew. Chem.* **1996,** *108*, 2554; Yang, Z. et al. *Angew. Chem.* **1997,** *109,* 170; Schinzer, D, et al. *Angew. Chem*. **1997,** *109,* 543).

Die Herstellung cyclischer Verbindungen aus offenkettigen Substraten ist ein zentrales Problem der chemischen Synthese. Häufig werden Cyclisierungen ausgehend von bi- oder polyfunktionellen Vorstufen in einer intramolekularen Ringschlußreaktion durchgeführt. Die gewünschten Ringschlußreaktionen stehen dabei prinzipiell in Konkurrenz zur Polymerisation der Substrate (unter Polymeren verstehen wir in diesem Zusammenhang Produkte die durch Verknüpfung zweier oder mehrerer Substratmoleküle entstanden sind, insbesondere auch Dimere sowie Oligomere niedrigen Molekulargewichts). Diese generelle Problematik gilt auch für die Herstellung cyclischer Produkte mittels Olefinmetathesen. Führt man diese Reaktion mit bi- oder polyfunktionellen Substraten durch, bei denen die genannten funktionellen Gruppen Alkene oder Alkine sind, so entstehen Gemische aus Cyclisierungsprodukt und Polymeren. In Schema l ist diese Konkurrenzsituation exemplarisch am Beispiel der Metathesereaktion des Diens **1** dargestellt.

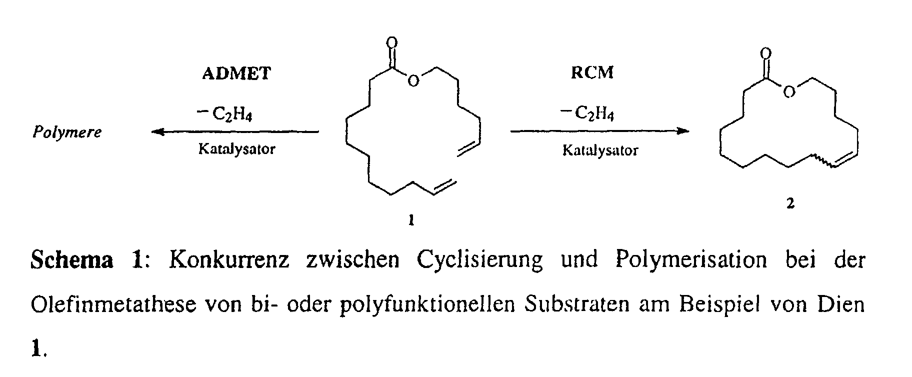

Die Produktverteilung zwischen Polymer und Cyclisierungsprodukt hängt im einzelnen von der Struktur der Substrate, dem verwendeten Katalysator sowie den Reaktionsbedingungen ab. Die Bildung des Cyclisierungsprodukts wird dabei durch die Durchführung der Reaktion in einem organischen Lösungsmittel bei hoher Verdünnung begünstigt. Dies gilt insbesondere für die Darstellung mittlerer (8-11 Ringglieder) und großer (≥ 12 Ringglieder) Ringe. Im allgemeinen werden für Olefinmetathesen Kohlenwasserstoffe (Hexan, Toluol, Xylol, Cumol etc. ) oder chlorierte Kohlenwasserstoffe (Dichlormethan, 1,2-Dichlorethan, Halogenbenzole etc.) als Lösungsmittel bevorzugt. Die zur Erreichung der notwendigen hohen Verdünnung erforderlichen großen Reaktionsvolumina bzw. aufwendigen Dosierverfahren limitieren die maximalen Raum-/Zeitausbeuten. Die Abtrennung der in hoher Verdünnung vorliegenden Produkte aus den Reaktionsgemischen erfordert ferner zeit- und energieaufwendigc Trennoperationen wie z. B. Chromatographie, Rektifikation oder Destillation. Die thermische Belastung bei destillativen Trennungen kann die Qualität der erhaltenen Produkte beeinträchtigen und führt nicht selten zu einer irreversiblen Desaktivierung der verwendeten Katalysatoren. Bei der Synthese von physiologisch aktiven Verbindungen stellen eventuell toxikologisch bedenkliche Lösungsmittelreste ein besonderes Problem dar. Auch hinsichtlich der möglichen Umweltbelastung birgt die Verwendung großer Lösungsmittelmengen prozeßtechnische Nachteile. Daher sind Verfahren, die zu einer vollständigen oder teilweisen Vermeidung der genannten Lösungsmittel führen, von hoher technischer Relevanz.

Kohlendioxid ist als umweltfreundliches Reaktionsmedium für metallkatalysierte Reaktionen vorgeschlagen worden [Übersichten: Jessop, P. G. et al. *Science* **1995,** *269,* 1065; Morgenstern, D. A. et al. in: *Green Chemistry* (Hrsg.: P. T. Anastas, T. C. Williamson) *ACS Symp. Ser. 262,* American Chemical Society, Washington DC, **1996,** S. 132ff; Dinjus, E. et al. in: *Chemistry under Extreme or Non-Classical Conditions,* (Hrsg.: R. van Eldik, C. D. Hubbard), Wiley, New York, **1996,** S. 219ff]. Metathesereaktionen in komprimiertem (gasförmigem, flüssigem oder überkritischem) Kohlendioxid werden in **WO 96/32421** beschrieben, sind jedoch dort ausschließlich mit ringöffnenden Polymerisationsreaktionen (ROMP) cyclischer monofunktioneller Alkene als Substrate belegt. Wir beschreiben nun ein Verfahren zur Herstellung chemischer Produkte durch selektive Olefinmetathese von bi- oder polyfunktionellen Substraten in Gegenwart eines homogen oder heterogen vorliegenden Metathesekatalysators in einem Reaktionsmedium, dadurch gekennzeichnet, daß die Substrate zwei oder mehrere funktionelle Gruppen in Form von substituierten oder unsubstituierten Alkenoder Alkin-Einheiten enthalten und das Reaktionsmedium im wesentlichen aus komprimiertem (gasförmigem, flüssigem oder überkritischem) Kohlendioxid besteht. Insbesondere betrifft die vorliegende Erfindung die Darstellung cyclischer Verbindungen mit Ringgrößen n≥5 durch Ringschlußmetathese bi- oder polyfunktioneller Substrate. Überraschenderweise finden wir, daß sich dabei durch Variation der Dichte des Reaktionsmediums wahlweise cyclische oder polymere Produkte mit hoher Selektivität gewinnen lassen.

Die Erfindung betrifft ein Verfahren zur Herstellung cyclischer Produkte durch selektive Olefinmetathese von bi- oder polyfunktionellen Substraten in Gegenwart von einem oder mehreren homogen oder heterogen vorliegenden Metathesekatalysatoren in einem Reaktionsmedium, dadurch gekennzeichnet, daß die Substrate zwei oder mehrere funktionelle Gruppen in Form von substituierten oder unsubstituierten Alken- oder Alkin-Einheiten enthalten und das Reaktionsmedium aus komprimiertem Kohlendioxid besteht, wobei die Reaktionstemperatur und der Gesamtdruck so aufeinander abgestimmt sind, daß die Dichte des Reaktionsmediums im Bereich von *d =* 0.4 - 1.5 g cm⁻³, bevorzugt im Bereich von *d =* 0.6-1.5 g cm⁻³ liegt.

Mithilfe der vorliegenden Erfindung können Carbo- und Heterocyclen beliebiger Ringröße n (n≥5), einschließlich der mittleren (n=8-11) und großen (n≥12) Ringe dargestellt werden. Die bislang in solchen Reaktionen verwendeten, zum Teil physiologisch bedenklichen und umweltschädlichen Lösungsmittel (z. B. Aromaten bzw. chlorierte Kohlenwasserstoffe) werden damit ganz oder großteils durch ein ungiftiges, nicht brennbares, billiges und wiederverwendbares Reaktionsmedium ersetzt. Die Reaktionsführung in komprimiertem Kohlendioxid führt ferner dazu, daß Susbtituenten an den Substraten toleriert werden, die mit der Olefinmetathese in konventionellen Lösungsmitteln nicht kompatibel sind. Ferner wird die Aufarbeitung der Reaktionsgemische aufgrund der speziellen Lösungseigenschaften von komprimiertem Kohlendioxid erheblich vereinfacht, beispielsweise indem die Produkte ganz oder teilweise aus der Reaktionsmischung abgeschieden werden, oder indem sie durch Ausnutzung der extraktiven Eigenschaften von komprimiertem CO₂ (K. Zosel, *Angew. Chem*. **1978,** *90,* 748) ganz oder teilweise vom Katalysator abgetrennt werden. Nach Abtrennung der Produkte sind die verbleibenden Katalysatoren zum Teil für Olefinmetathesen wiederverwendbar.

Die Reaktionstemperatur und der Gesamtdruck können bei der selektiven Olefinmetathese bi- oder polyfunktioneller Substrate in komprimiertem Kohlendioxid in weiten Bereichen frei gewählt werden, sind jedoch so aufeinander abgestimmt, daß die Dichte des Reaktionsmediums im Bereich von *d* = 0.2 - 1.5 g cm⁻³ liegt. Bevorzugte Reaktionstemperaturen liegen im Bereich von 250 - 400 K, besonders bevorzugt im Bereich von 280 - 345 K. Bevorzugte Gesamtdrücke liegen im Bereich von 30 - 300 bar, besonders bevorzugt im Bereich von 50 - 220 bar. Die Ringschlußreaktion des bi- oder polyfunktionellen Substrats wird dabei durch Anwendung hoher Dichten begünstigt, während die Polymerisation im Bereich niedriger Dichten bevorzugt abläuft. Der jeweils optimale Dichtebereich ist dabei von der Struktur des Substrats und vom verwendeten Katalysator abhängig.

Die Ringschlureaktion erfolgt gemäß der vorliegenden Erfindung bevorzugt bei Dichten *d =* 0.4-1.5 g cm⁻³, besonders bevorzugt bei Dichten *d =* 0.6-1.5 g cm⁻³. Hohe Dichten werden insbesondere für die Darstellung mittlerer und großer Ringe bevorzugt (für das spezielle Beispiel der Metathese von Dien **1** zu Lacton **2** siehe Abbildung 1)., während für die Darstellung von Ringen mit n=5-7 die Wahl der Dichte weniger kritisch ist. Besonders bevorzugte Bedingungen für die Ringschlußreaktion sind aus den angefügten Beispielen ersichtlich, sollen die Anwendbarkeit der vorliegenden Erfindung jedoch in keiner Weise einschränken.

Im Gegensatz zur Cyclisierung erfolgt die Polymerisation durch Metathese eines bi- oder polyfunktionellen Substrats bevorzugt bei niedrigen Dichten *d* = 0.2-0.65 g cm⁻³ des im wesentlichen aus komprimiertem Kohlendioxid bestehenden Reaktionsmediums. Wie für die RCM hängt der optimale Dichtebereich von Substrat und Katalysator ab. Für das spezielle Beispiel der Metathese unter Polyemerisation des Diens **1** siehe Abbildung 1.

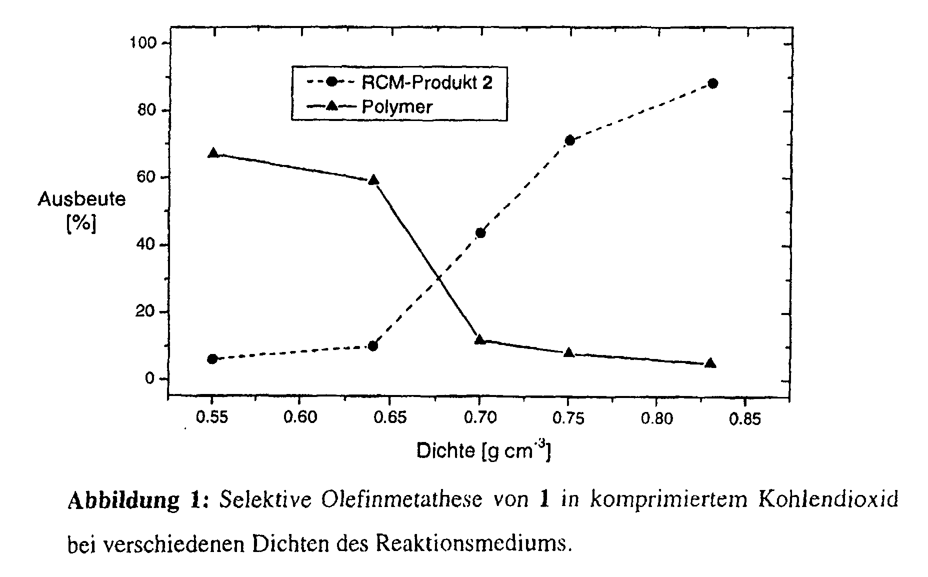

Als Katalysatoren bzw. Katalysator-Vorstufen kommen in der vorliegenden Erfindung alle metatheseaktiven Metallverbindungen in Betracht, unabhängig davon ob sie im Reaktionsmedium homogen oder heterogen vorliegen, sofern sie nicht von komprimiertem Kohlendioxid inaktiviert werden. Die Katalysatoren können in isolierter Form eingesetzt oder in situ im Reaktionsmedium aus geeigneten Vorläufern erzeugt werden. Die eingesetzte Katalysatormenge ist nicht kritisch, wobei bevorzugte Katalysatormengen im Bereich von 0.01 - 10 mol% bezogen auf das eingesetzte Substrat liegen. Als bevorzugte Katalysatoren bzw. Katalysator-Vorstufen dienen Übergangsmetall-Carbene oder Übergangsmetallverbindungen, die unter den Reaktionsbedingungen Metall-Carbene bilden, oder Übergangsmetallsalze in Verbindung mit einem Alkylierungsmittel. Dazu zählen unter anderem Systeme der allgemeinen Typen **I-IX** (Literatur: Typ **I** (M = Ru, Os): **WO** 96/04289, 15.02.1996; Nguyen et al. *J. Am. Chem. Soc.* **1992,** *114,* 3974; Nguyen et al. *J. Am. Chem. Soc.* **1993,** *115*, 9858; Schwab, P. et al. *Angew. Chem*. **1995,** *107*, 2179; Schwab, P. et al. *J. Am. Chem. Soc.* **1996,** *118,* 100; Mohr, B. et al. *Organometallics* **1996,** *15*, 4317. Typ **II** (M = Mo, W): Schrock, R. R. et al. *J. Am. Chem. Soc.* **1990,** *112*, 3875; Fujimura, O. et al. *Organometallics* **1996,** *15*, 1865. Typ **III:** Quingnard, F. et al. *J. Mol. Catal*. **1986,** *36*, 13. Typ **IV** (M = Nb, Ta): Rocklage, S. M. et al. *J. Am. Chem. Soc.* **1981,** *103,* 1440; Wallace, K. C. et al. *Macromolecules* **1987,** *20,* 448. Typ **V** (cp = substituiertes oder unsubstituiertes Cyclopentadienyl): **US** 4,567,244, 28.01.1986. Typ **VI:** Herrmann, W. A. et al. *Angew. Chem.* **1991,** *103*, 1704. Typ **VII:** Nugent, W. A. et al. *J. Am. Chem. Soc.* **1995,** *117*, 8992. Typ **VIII:** Davie, E. S. *J. Catal*. **1972,** *24*, 272. Typ **IX:** Herrmann, W. A. et al. *Angew. Chem.* **1996,** *108*, 1169.) 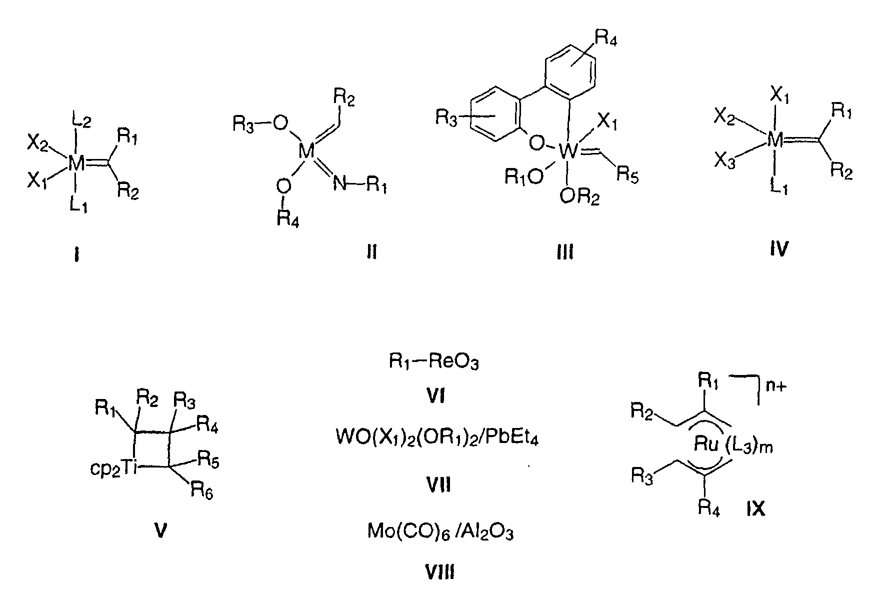 R₁-R₆ sind unabhängig voneinander wählbare Reste aus Wasserstoff, C₂-C₂₀ Alkenyl, C₂-C₂₀ Alkinyl, C₁-C₂₀ Alkyl, Aryl, C₁-C₂₀ Carboxylat, C₁-C₂₀ Alkoxy, C₂-C₂₀ Alkenyloxy, C₂-C₂₀ Alkinyloxy, Aryloxy, C₂-C₂₀ Alkoxycarbonyl, C₁-C₂₀ Alkylthio, C₁-C₂₀ Alkylsulfonyl oder C₁-C₂₀ Alkylsufinyl; jeweils wahlweise substituiert mit C₁-C₁₂ Alkyl, C₁-C₁₂ Perfluoralkyl, Halogen, C₁-C₅ Alkoxy, oder Aryl. Die Reste R₁-R₆ können in cyclischen Verbindungen miteinander verknüpft vorliegen.

X₁-X₃ sind unabhängig voneinander wählbare anionische Liganden beliebiger Definition, insbesondere wählbar aus F⁻, Cl⁻, Br-, I⁻, CN⁻, SCN⁻, R₁O⁻, R₁R₂N⁻, (R₁-R₅)-Allyl⁻, (R₁-R₅)-Cyclopentadienyl⁻, wobei die Reste R₁-R₅ die bereits genannte Definition erfüllen.

L₁-L₃ sind unabhängig voneinander wählbare neutrale Liganden, insbesondere wählbar aus CO, CO₂, R₁NCO, R₁R₂C=CR₃R₄, R₁C≡CR₂, R₁R₂C=NR₃, R₁C≡N, R₁OR₂, R₁SR₂, NR₁R₂R₃, PR₁R₂R₃, AsR₁R₂R₃, SbR₁R₂R₃, wobei die Reste R₁-R₄ die bereits genannte Definition erfüllen.

m, n sind ganze Zahlen zwischen 0 und 6.

Besonders bevorzugte Katalysatoren oder Katalysator-Vorstufen sind Carben-Komplexe des allgemeinen Typs I mit L₁-L₂ = PR₁R₂R₃, wobei R₁-R₃ den oben genannten Definitionen entsprechen; speziell bevorzugte Reste R₁-R₃ sind dabei Aryl oder Alkyl, insbesondere sekundäre Alkyl oder Cycloalkyreste. Ebenso sind besonders bevorzugt Carbenkomplexe des allgemeinen Typs **II** mit R₁ = Aryl und R₂- R₄ = C₁-C₂₀ Alkyl, jeweils wahlweise substituiert mit C₁-C₁₂ Alkyl, C₁-C₁₂ Perfluoralkyl, Aryl, Halogen.

Reaktionen gemäß der vorliegenden Erfindung können auch in Gegenwart von einem odere mehrerer Additiva durchgeführt werden, wodurch zum Beipiel eine leichtere Handhabung der Substrate oder Katalysatoren, oder eine Verbesserung der Lösungseigenschaften des Reaktionsmediums, oder eine Erhöhung der Reaktionsgeschwindigkeit oder eine Verbesserung der Ausbeute resultieren kann. Solche Additiva können beispielsweise unabhängig gewählt werden aus: Wasser, Phosphorverbindungen, Amine, perfluorierte Verbindungen (siehe Patentanmeldung Studiengesellschaft Kohle m.b.H. DE 197 02 025.9, 23.1.97), Lewis-acide Verbindungen, Metallalkoxide, organische Lösungsmittel (z. B. Dichlormethan, Trichlormethan, Tetrachlormethan, 1,2-Dichlorethan, Trichlorethen, Benzol, Toluol, Xylol, Cumol, Hexan, Cyclohexan, Halogenbenzole, Tetrahydrofuran, *tert*-Butylmethylether, Diethylether, Dimethoxyethan, Dimethylformamid, Acetessigester, Aceton, Dimethylcarbonat, Alkohole).

Als bi- oder polyfunktionelle Substrate werden in der vorliegenden Erfindung alle chemischen Verbindungen bezeichnet, die zwei oder mehrere funktionelle Gruppen in Form von substituierten oder unsubstituierten Alken- oder Alkin-Einheiten enthalten, welche die Metathesereaktion erlauben. Die Substrate können entweder konformativ prä-organisiert oder aber vollkommen flexibel sein. Neben den genannten an der Reaktion teilnehmenden funtionellen Gruppen können die Substrate eine beliebige Anzahl weiterer, mit der Metathesereaktion kompatibler Gruppen oder Heteroatome enthalten. Dies umfaßt unter anderem verzweigte oder unverzweigte Alkylreste, aromatische oder nicht-aromatische carbocyclische Ringe, Carbonsäuren, Ester, Ether, Epoxide, Silylether, Thioether, Thioacetale, Anhydride, Imine, Silylenolether, Ammoniumsalze, Amide, Nitrile, Perfluoralkyl-Gruppen, *gem*-Dialkyl-Gruppen, Alkine, Alkene, Halogene, Alkohole, Ketone, Aldehyde, Carbamate, Carbonate, Urethane, Sulfonate, Sulfone, Sulfonamide, Nitro-Gruppen, Organosilan-Einheiten, Metallzentren, sauerstoff-, stickstoff-, schwefel-, phosphorhaltige Heterocyclen. Es können auch Mischungen von Substraten gemäß der vorliegenden Erfindung umgesetzt werden, wobei die Substrate dabei im Gemisch oder sequentiell dem Reaktionsmedium zugeführt werden können. Die Substrate können auch in trägergebundener Form vorliegen.

Insbesondere sind selektive Olefinmetathesen in komprimiertem Kohlendioxid auch mit bi- oder polyfunktionellen Substraten durchführbar, die eine oder mehrere primäre, sekundäre oder tertiäre basische Amin-Einheiten enthalten, welche in konventionellen Lösungsmitteln zur Desaktivierung der Metathesekatalysatoren führen können.

Durch selektive Olefinmetathese bi- oder polyfunktioneller Substrate in komprimiertem Kohlendioxid sind insbesondere makrocyclische Ester, Ether, Amine und Ketone erhältlich, die z. B. als Geruchstoffe und Vorstufen davon Verwendung finden können. Die Anwendungbreite beinhaltet unter anderem jene der RCM in organischen Lösungsmitteln (siehe u.a. US Patentanmeldung 08/767.561 vom 16.12.1996, Studiengesellschaft Kohle mbH). Dies umfaßt beispielsweise die makrocyclischen Ester **2** und **4** und Doppelbindungsisomere davon (z. B. aus Substraten **1** und **3**), die selbst über einen ausgeprägt moschusartigen Geruch verfügen und durch Hydrierung in die bekannten Parfuminhaltsstoffe Pentadecanolid bzw. Arova 16® überführt werden.

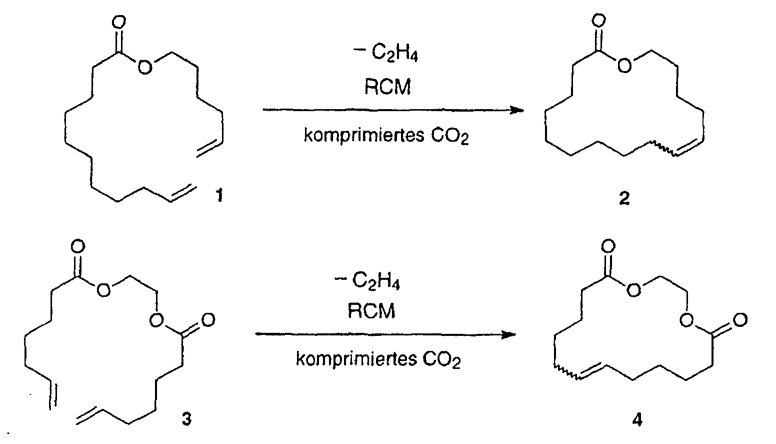

Die Anwendungsbreite der selektive Olefinmetathese bi- oder polyfunktioneller Substrate in komprimiertem Kohlendioxid umfaßt ferner eine homologe Reihe substituierter oder unsubstituierter cyclischer Ketone mit Ringgrößen von 12-30, einschließlich von Zibeton, Muscon, Exalton® und Muscenon® (Übersicht: Ohloff, G. *Riechstoffe und Geruchssinn,* Springer, Berlin, **1990;** Bauer K. et al., *Ullmann's Encyclopedia of Industrial Chemistry,* VCH, Weinheim, 5th Ed., **1988,** Vol. A11, 141). Der Antitumorwirkstoff Epothilon und seine Analoga lassen sich ebenfalls nach diesem Reaktionsprinzip darstellen (Bertinato, P. et al. *J. Org. Chem.* **1996,** *61,* 8000; Nicolaou, K. C. et al. *Angew. Chem.* **1996,** *108*, 2554; Yang, Z. et al. *Angew. Chem*. **1997,** *109,* 170; Schinzer, D. et al. *Angew. Chem.* **1997,** *109*, 543).

Die durch selektive Olefinmetathese bi- oder polyfunktioneller Substrate in komprimiertem Kohlendioxid dargestellten Verbindungen werden beim Entspannen des Reaktors ganz oder teilweise vom Katalysator abgetrennt und lassen sich in geeigneten Vorlagen sammeln. Das bei der Isolierung der Produkte freiwerdende Kohlendioxid kann erneut zur Beschickung bzw. zum Spülen des Reaktors herangezogen werden. Ferner kann der auf diese Weise vom Produkt abgetrennte Katalysator für Metathesereaktionen wiederverwendet werden.

An selektive Olefinmetathesen bi- oder polyfunktioneller Substrate in komprimiertem Kohlendioxid können sich in integrierten Verfahren auch weitere Umsetzungen in komprimiertem Kohlendioxid wie z.B. Kohlenstoff-Kohlenstoff-Verknüpfung, Reduktion oder Oxidation zur weiteren Funktionalisierung der gewonnen Produkte anschließen. Insbesondere lassen sich durch selektive Olefinmetathese und nachgeschaltete Hydrierung im komprimiertem Kohlendioxid als Reaktionsmedium gesättigte macrocyclische Moschusriechstoffe wie beispielsweise Exaltolid®, Arova 16® und Exalton® direkt aus geeigneten acyclischen ungesättigten Vorstufen in einem integrierten Verfahren gewinnen.

Durch selektive Olefinmetathese bi- oder polyfunktioneller Substrate in komprimiertem Kohlendioxid lassen sich ungesättigte Polymere wie z.B. polymere Kohlenwasserstoffe, Polyester, Polyamide, Polycarbonate, Polyurethane und dergleichen darstellen. Diese haben wirtschaftliche Bedeutung u.a. als Thermoplasten, Elastomere, Füllstoffe, Dämm-Materialien, Adsorber sowie in der Herstellung von optischen Bauteilen und als Zusatzstoffe in der Vulkanisation.

Die aus der Reaktionsmischung anfallenden Polymere können mit Hilfe gängiger Methoden weiter gereinigt und verarbeitet werden. Weitere Umsetzungen der Polymere wie Hydrierung oder Oxidation können ebenso wie bekannte Methoden zum Polymerprocessing in komprimiertem Kohlendioxid [Schmeder, H. in: *Chemistry under Extreme or Non-Classical Conditions*, (Hrsg.: R. van Eldik, C. D. Hubbard), Wiley, New York, **1996,** S. 280f] im Rahmen integrierter Prozesse Anwendung finden. Das beim Entspannen freiwerdende Kohlendioxid kann erneut komprimiert und als Reaktionsmedium eingesetzt werden.

Die im folgenden angeführten Beispiele beschreiben prototypischc Metathesereaktionen in komprimiertem Kohlendioxid unter bevorzugten Bedingungen, sollen jedoch in keiner Weise den Umfang, die Anwendungsbreite oder Vorteile der vorliegenden Erfindung einschränken. Die Abkürzung Cy steht für Cyclohexyl (cyclo-C₆H₁₁), *d* für Dichte und cat. für Katalysator.

### BEISPIEL 1

### Darstellung von Oxacyclohexadec-11-en-2-on (2).

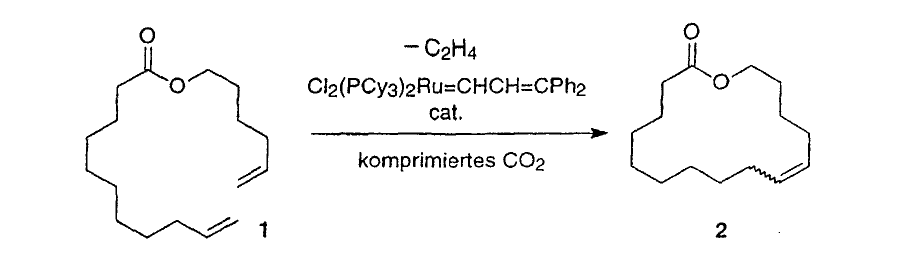

Das Dien **1** (180 mg) und *trans*-[Cl₂(PCy₃)₂Ru=CHCH=CPh₂] (7 mg) wurden unter Argon-Atmosphäre in einen Edelstahl-Hochdruckreaktor (*V* = 225 cm⁻³) eingebracht. Dieser wurde mit 170 g CO₂ mittels eines Kompressors befüllt (*d* = 0.76 g cm⁻³) und das Reaktionsgemisch 72 h bei 313 K gerührt. Anschließend wurde das CO₂ über eine auf 233 K gekühlte Kühlfalle entspannt. Zur vollständigen Entfernung des Produkts aus dem Reaktor wurde noch zweimal mit je 170 g CO₂ gespült und wiederum über die gekühlte Kühlfalle entspannt. Nach Aufwärmen wurden aus der Kühlfalle 157 mg eines farblosen Öls erhalten, das laut GC-Analyse zu 71 % aus Verbindung **2** als Mischung der Doppelbindungs-Isomere (cis:trans = 26:74) und zu 21 % aus unumgesetzten **1** bestand.
¹H NMR (200 MHz, CDCl₃) δ 5.45 - 5.28 (m, 2H), 4.18 - 4.07 (m, 2H), 2.37 - 2.29 (m, 2H), 2.10 - 2.00 (m, 4H), 1.72 - 1.54 (m, 4H), 1.49 - 1.30 (m, 10H); IR (Film) 3000, 2928, 2856, 1736, 1461, 1385, 1346, 1252, 1234, 1168, 1152, 1113, 1085, 1024, 969, 719 cm⁻¹.

### BEISPIEL 2

### Selektive Olefinmetathese von Alken (1) zur Darstellung von (2) oder von Polymeren bei verschiedenen Dichten der Reaktionsmischung

Das Dien **1** (180 mg) und *trans*-[Cl₂(PCy₃)₂Ru=CHCH=CPh₂] (7 mg) wurden unter Argon-Atmosphäre in einen Edelstahl-Hochdruckreaktor (*V* = 225 cm⁻³) eingebracht. Dieser wurde mit der gewünschten Menge CO₂ mittels eines Kompressors befüllt und das Reaktionsgemisch 72 h bei 313 K gerührt. Anschließend wurde das CO₂ über eine auf 233 K gekühlte Kühlfalle entspannt und der Reaktor wurde mit Aceton gespült. Die vereinigten Fraktionen wurden zur Trockne eingeengt und durch Chromatographie an Kieselgel mit Hexan/Essigester als Eluens wurden die niedrigemolekularen Bestandteile (unumgesetztes **1** und Cyclisierungsprodukt **2**) von den Oligomeren abgetrennt. Die Anteile von **1** und **2** wurden mittels GC-Analyse bestimmt. Die Ergebnisse sind in Tabelle 1 zusammengefaßt und in Abbildung 1 graphisch dargestellt.

**Tabelle 1:**

| Selektive Olefinmetathese von **1** in komprimiertem Kohlendioxid bei verschiedenen Dichten des Reaktionsmediums | | | | | |
|---|---|---|---|---|---|
| Beispiel | CO₂ [g] | d[g cm⁻³] | 2[%] | Polymer [%] | _{**1**}a[%] |
| 2a | 186 | 0.83 | 88.3 | 5 | 9 |
| 2b | 170 | 0.76 | 71.1 | 8 | 21 |
| 2c | 157 | 0.70 | 43.5 | 12 | 44 |
| 2d | 145 | 0.64 | 10 | 59 | 31 |
| 2e | 124 | 0.55 | 6 | 67 | 27 |

| | | | | | |
|---|---|---|---|---|---|
| ^{a}) nach Beendigung der Reaktion unumgesetztes Substrat. | | | | | |

### BEISPIEL 3

### Darstellung von 1,4-Dioxacyclohexadec-10-en-5,16-dion (4) und Wiederverwendbarkeit des Katalysators

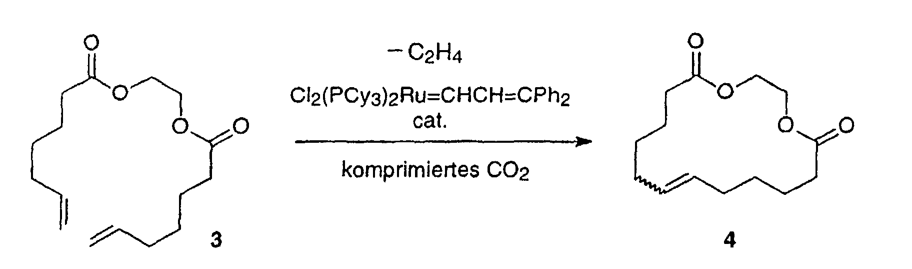

Der ungesättigte Ester 3 (180 mg) und *trans*-[Cl₂(PCy₃)₂Ru=CHCH=CPh₂] (14 mg) wurden unter Argon-Atmosphäre in einen Edelstahl-Hochdruckreaktor (*V* = 225 cm⁻³) eingebracht. Mittels eines Kompressors wurde der Reaktor mit 140 g CO₂ befüllt (*d*=0.62 g cm⁻³) und das entstehende Reaktionsgemisch 72 h bei 313 K gerührt. Das CO₂ wurde über eine auf 233 K gekühlte Kühlfalle entspannt.

Nach Aufwärmen wurden aus der Kühlfalle 160 mg eines farblosen Öls erhalten, das laut GC-Analyse zu 30.4 % aus Verbindung **4** bestand (Bsp. 4a).

Der im Reaktor verbliebene Rückstand wurde erneut mit **3** (170 mg) und CO₂ (140 g) beladen (*d* = 0.62 g cm⁻³) und 170 h bei 313 K gerührt. Nach Entspannen wie unter Bsp. 4a beschrieben wurden die Kühlfalle und der Reaktor mit CH₂Cl₂ gespült und die vereinigten Waschlösungen eingeengt. Man erhielt 160 mg eines farblosen Öls, das laut GC zu 70.4 % aus **4** bestand. Das erhaltene Rohprodukt wurde durch Säulenchromatographie gereinigt. Verbindung **4** fiel in Form farbloser Kristalle an.
**Ausbeute:** 102 mg (67 %)
Schmp 46 - 47°C; ¹H NMR (200 MHz, CDCl₃) δ 5.39 - 5.21 (m, 2H), 4.30 (s, 1H), 4.27 (s, 3H), 2.37 - 2.26 (m, 4H), 2.11 - 2.02 (m, 4H), 1.71 - 1.55 (m, 4H), 1.48 - 1.38 (m, 4H); IR (KBr) 2931, 2854, 1733, 1462, 1439, 1398, 1371, 1296, 1275, 1257, 1223, 1169, 1102, 1072, 1035, 965, 874 cm⁻¹.

### BEISPIEL 4

### Darstellung von 2,2,5-Trimethylcyclo-4-heptenon (6).

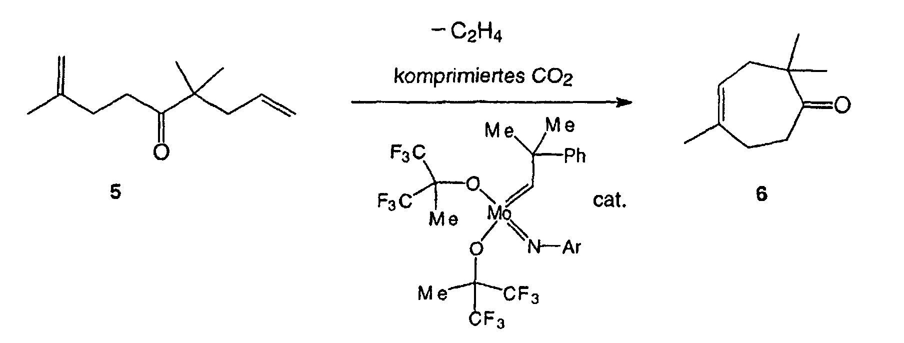

Das Dienon **5** (222 mg) und [{(CF₃)₂MeCO}₂Mo(=CHCMe₂Ph)(=NC₆H₃-*i*-Pr₂-2,6)] (46 mg) wurden unter Argon-Atmosphäre in einen Edelstahl-Hochdruckreaktor (*V* = 225 cm⁻³) eingebracht. Der Reaktor wurde mittels eines Kompressors mit 170 g CO₂ befüllt (*d* = 0.76 g cm⁻³) und das entstehende Reaktionsgemisch bei 313 K gerührt. Nach 72 h wurde das CO₂ über eine auf 233 K gekühlte Kühlfalle entspannt. Der Reaktor und die Kühlfalle wurden mit Aceton gespült, die vereinigten Waschlösungen eingeengt und der verbleibende Rückstand säulenchromatographisch (Kieselgel, Hexan/Essigester 12:1) gereinigt. Das Produkt **6** (117 mg, 62 %) und umumgesetztes **5** (56 mg, 25 mg) wurden als farblose Öle erhalten.

### BEISPIEL 5

### Darstellung von 3,3'-Bis-2,5-Dihydrofuranyl (8).

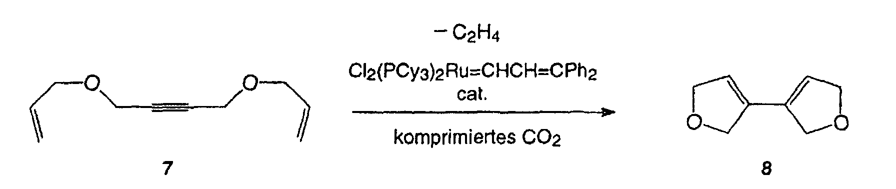

Der ungesättigte Ester **7** (187 mg) und *trans-*[Cl₂(PCy₃)₂Ru=CHCH=CPh₂] (19 mg) wurden unter Argon-Atmosphäre in einen Edelstahl-Hochdruckreaktor (*V* = 225 cm⁻³) eingebracht. Dieser wurde mit 169 g CO₂ mittels eines Kompressors befüllt (*d* = 0.75 g cm⁻³) und das Reaktionsgemisch 48 h bei 313 K gerührt. Anschließend wurde das CO₂ über eine auf 233 K gekühlte Kühlfalle entspannt und der Reaktor wurde mit Aceton gespült. Die vereinigten Fraktionen wurden zur Trockne eingeengt und nach Chromatographie an Kieselgel mit Hexan/Essigester (10:1) wurde reines **8** (97 mg, 62 %) als farbloser Feststoff erhalten.

### BEISPIEL 6

### Darstellung von 1-(Toluene-4-sulfonyl)-2,5-dihydro-1H-pyrrol (10).

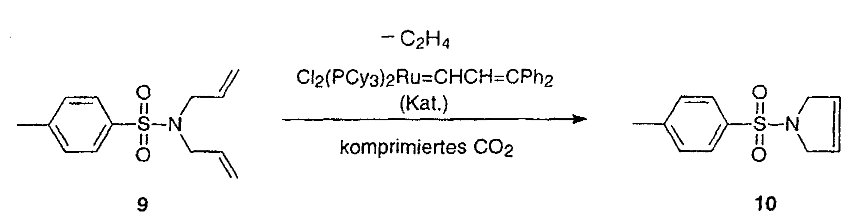

Das Dien **9** (219 mg) und *trans*-[Cl₂(PCy₃)₂Ru=CHCH=CPh₂] (8 mg) wurden unter Argon-Atmosphäre in einen Edelstahl-Hochdruckreaktor (*V* = 225 cm⁻³) eingebracht. Der Reaktor wurde mittels eines Kompressors mit 170 g CO₂ befüllt (*d* = 0.76 g cm⁻³) und das entstehende Reaktionsgemisch bei 313 K gerührt. Nach 24 h wurde das CO₂ über eine auf 233 K gekühlte Kühlfalle entspannt. Der Reaktor und die Kühlfalle wurden mit Aceton gespült, die vereinigten Waschlösungen eingeengt und der verbleibende Rückstand säulenchromatographisch gereinigt. Das Produkt **10** wurde in Form farbloser Kristalle erhalten.
**Ausbeute**: 175 mg (93 %)
Schmp. 123 - 124°C; 'H NMR (200 MHz, CDCl₃) δ 7.73 (d, 2H, J = 8.3), 7.32 (d, 2H, J = 8.0), 5.67 (t, 2H, J = 4.6), 4.12 (t, 4H, J = 4.5), 2.43 (s, 3H); IR (Film) 3049, 2910, 2854, 1595, 1493, 1476, 1337, 1306, 1162, 1112, 1071, 1018, 1002, 948, 820, 710, 667, 601, 564 cm⁻¹.

### BEISPIEL 7

### Darstellung von 1-(4-Bromphenyl)-cyclopent-3-enol (12).

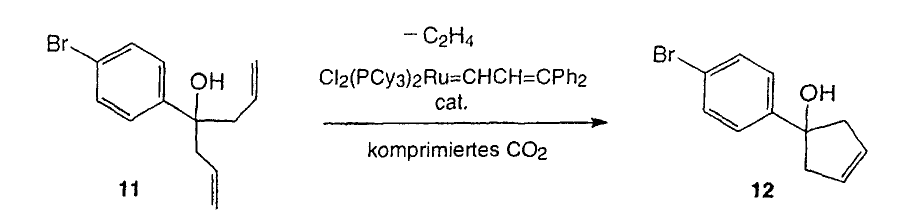

Das Hydroxy-Dien **11** (345 mg) und *trans*-[Cl₂(PCy₃)₂Ru=CHCH=CPh₂] (9 mg) wurden unter Argon-Atmosphäre in einen Edelstahl-Hochdruckreaktor (*V* = 225 cm⁻³) eingebracht. Mittels eines Kompressors wurde der Reaktor mit 177 g CO₂ befüllt (*d* = 0.79 g cm⁻³) und das entstehende Reaktionsgemisch 18 h bei 313 K gerührt. Das CO₂ wurde über drei auf 233 K gekühlte Kühlfallen entspannt, und der Reaktor noch zweimal mit je 170 g CO₂ nachgespült. Nach Aufwärmen wurden aus den Kühlfallen 87 mg eines farblosen Feststoffs erhalten. Aus dem im Reaktor verbliebenen Rückstand konnten mittels Aceton weitere 134 mg Rohprodukt der gleichen Zusammensetzung isoliert werden. Säulenchromatographische Reinigung der vereinigten Fraktionen ergab **12** als farblosen Feststoff.
**Ausbeute:** 99 mg (32 %)
Schmp 77 - 78°C; ¹H NMR (200 MHz, CDCl₃) δ 7.50 - 7.34 (m, 4H), 5.85-5.76 (m, 2H), 2.89 (d, 2H, *J =* 16.0 Hz), 2.71 (d, 2H, *J* = 16.0 Hz), 2.15 (s, 1H); IR (KBr) 3331, 3059, 2911, 2846, 1904, 1658, 1614, 1589, 1483, 1426, 1399, 1332, 1306, 1270, 1159, 1073, 1010, 977, 876, 826, 777, 702, 668, 542 cm⁻¹.

### BEISPIEL 8

**Darstellung von Epilachnen (14)**.

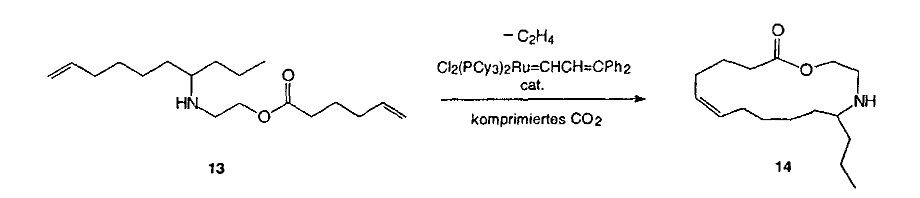

Das Aminodien **13** (52 mg, gelöst in 0.5 ml CH₂Cl₂) und *trans*-[Cl₂(PCy₃)₂Ru=CHCH=CPh₂] (7 mg) wurden unter Argon-Atmosphäre in einen Edelstahl-Hochdruckreaktor (*V* = 225 cm⁻³) eingebracht. Mittels eines Kompressors wurde der Reaktor mit 172 g CO₂ (*d* = 0.76 g cm⁻³) befüllt und das entstehende Reaktionsgemisch 72 h bei 313 K gerührt. Das CO₂ wurde über eine auf 233 K gekühlte Kühlfallen entspannt, und der Reaktor mit Et₂O nachgespült. Säulenchromatographische Reinigung (Aluminiumoxid, Hexan/Essigsester 1:1) der vereinigten Fraktionen ergab 44 mg eines schwach gelblichen Öls, das laut GC-Analyse zu 74 % aus **14** als Mischung der Doppelbindungs-Isomeren (cis:trans = 30:70) und zu 22 % aus unumgesetzten **13** bestand.

## Patentansprüche

1. Verfahren zur Herstellung cyclischer Produkte durch selektive Olefinmetathese von bi- oder polyfunktionellen Substraten in Gegenwart von einem oder mehreren homogen oder heterogen vorliègenden Metathesekatalysatoren in einem Reaktionsmedium, **dadurch gekennzeichnet, daß** die Substrate zwei oder mehrere funktionelle Gruppen in Form von substituierten oder unsubstituierten Alken- oder Alkin-Einheiten enthalten und das Reaktionsmedium aus komprimiertem Kohlendioxid besteht, wobei die Reaktionstemperatur und der Gesamtdruck so aufeinander abgestimmt sind, daß die Dichte des Reaktionsmediums im Bereich von *d* = 0.4 - 1.5 g cm⁻³, bevorzugt im Bereich von *d* = 0.6-1.5 g cm⁻³ liegt.

2. Verfahren nach Anspruch 1, wobei die Produkte carbo- oder heterocyclische Verbindungen mit Ringgrößen von ≥ 5 darstellen.

3. Verfahren nach Anspruch 2, wobei die Produkte carbo- oder heterocyclische Verbindungen mit Ringgrößen von 8-11 darstellen.

4. Verfahren nach Anspruch 2, wobei die Produkte makrocyclische Verbindungen mit Ringgrößen von ≥ 12 darstellen, insbesondere von Pentadecanolid und homologen Verbindungen, Dodecandisäureethylester und homologen Verbindungen, Cycloheptadec-9-en-1-on und homologen Verbindungen, 3-Methyl-cyclopentadecan-1-on und homologen Verbindungen, Cyclopentadecanon und homologen Verbindungen, 3-Methyl-cyclopentadec-5-en-1-on und homologen Verbindungen, Epothilon und homologen Verbindungen.

5. Verfahren zur Herstellung polymerer (einschließlich dimerer oder oligomerer) Produkte durch selektive Olefinmetathese von bi- oder polyfunktionellen Substraten in Gegenwart eines homogen oder heterogen vorliegenden Metathesekatalysators in einem Reaktionsmedium, **dadurch gekennzeichnet, daß** die Substrate zwei oder mehrere funktionelle Gruppen in Form von substituierten oder unsubstituierten Alken- oder Alkin-Einheiten enthalten und das Reaktionsmedium aus komprimiertem Kohlendioxid besteht, wobei die Reaktionstemperatur und der Gesamtdruck so aufeinander abgestimmt sind, daß die Dichte des Reaktionsmediums im Bereich von *d* = 0.2 - 1.3 g cm⁻³, bevorzugt im Bereich von *d* = 0.2 - 0.65 g cm⁻³.

6. Verfahren nach Anspruch 1-5, wobei als Katalysatoren oder Katalysator-Vorstufen metatheseaktiven Metallverbindungen verwendet werden, die nicht von komprimiertem Kohlendioxid inaktiviert werden.

7. Verfahren nach Anspruch 6, wobei als Katalysatoren oder Katalysator-Vorstufen Übergangsmetall-Carbene oder Übergangsmetallverbindungen, die unter den Reaktionsbedingungen Metall-Carbene bilden, oder Übergangsmetallsalze in Verbindung mit einem Alkylierungsmittel verwendet werden.

8. Verfahren nach Anspruch 7, wobei als Katalysatoren oder Katalysator-Vorstufen Vcrbindungen der allgemeinen Typen **I-IX** verwendet werden; 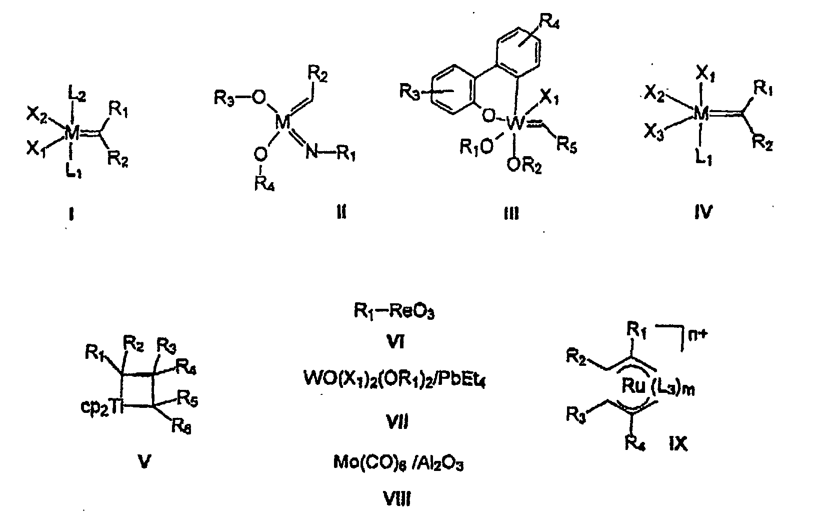
R₁-R₆ sind unabhängig voneinander wählbare Reste aus Wasserstoff, C₂-C₂₀ Alkenyl, C₂-C₂₀ Alkinyl, C₁-C₂₀ Alkyl, Aryl, C₁-C₂₀ Carboxylat, C₁-C₂₀ Alkoxy, C₂-C₂₀ Alkenyloxy, C₂-C₂₀ Alkinyloxy, Aryloxy. C₂-C₂₀ Alkoxycarbonyl, C₁-C₂₀ Alkylthio, C₁-C₂₀ Alkylsulfonyl oder C₁-C₂₀ Alkylsufinyl; jeweils wahlweise substituiert mit C₁-C₁₂ Alkyl, C₁-C₁₂ Perfluoralkyl, Halogen, C₁-C₅ Alkoxy, oder Aryl; die Reste R₁-R₆ können in cyclischen Verbindungen miteinander verknüpft vorliegen;
X₁-X₃ sind unabhängig voneinander wählbare anionische Liganden, insbesondere wählbar aus F⁻, Cl⁻, Br⁻, I⁻, CN⁻, SCN⁻, R₁O⁻, R₁R₂N⁻, (R₁-R₅)-allyl⁻, (R₁-R₅)-Cyclopentadienyl⁻, wobei die Reste R₁-R₅ die bereits genannte Definition erfüllen;
L₁-L₃ sind unabhängig voneinander wählbare neutrale Liganden, insbesondere wählbar aus CO, CO₂, R₁NCO, R₁R₂C=CR₃R₄, R₁C≡CR₂, R₁R₂C=NR₃, R₁C≡N, R₁OR₂, R₁SR₂, NR₁R₂R₃, PR₁R₂R₃, AsR₁R₂R₃, SbR₁R₂R₃;
m und n sind ganze Zahlen zwischen 0 und 6;

9. Verfahren nach Anspruch 8, wobei als Katalysatoren oder Katalysator-Vorstufen Carben-Komplexe des allgemeinen Typs **I** mit L₁-L₂ = PR₁R₂R₃ verwendet werden, in denen R₁-R₃ den oben genannten Definitionen entsprechen; bevorzugte Reste R₁-R₃ sind dabei Aryl oder Alkyl, insbesondere sekundäre Alkyl oder Cycloalkyreste; ebenso sind bevorzugt Carbenkomplexe des allgemeinen Typs **II** mit R₁ = Aryl und R₂-R₄ = C₁-C₂₀ Alkyl, jeweils wahlweise substituiert mit C₁-C₁₂ Alkyl, C₁-C₁₂ Perfluoralkyl, Aryl, Halogen.

10. Verfahren nach Anspruch 1-9, wobei die bi- oder polyfunktionellen Substrate konformativ prä-organisiert oder vollkommen flexibel sind.

11. Verfahren nach Anspruch 1-10, wobei die bi- oder polyfunktionellen Substrate neben den an der Reaktion teilnehmenden funtionellen Gruppen eine beliebige Anzahl weiterer, mit der Metathesereaktion kompatibler Gruppen oder Heteroatome enthalten.

12. Verfahren nach Anspruch 11, wobei die genannten Gruppen oder Heteroatome unabhängig gewählt werden aus: verzweigte oder unverzweigte Alkylreste, aromatische oder nicht-aromatische carbocyclische Ringe, Carbonsäuren, Ester, Ether, Epoxide, Silylether, Thioether, Thioacetale, Anhydride, Imine, Silylenolether, Ammoniumsalze, Amide, Nitrile, Perfluoralkyl-Gruppen, *gem-*Dialkyl-Gruppen, Alkine, Alkene, Halogene, Alkohole, Ketone, Aldehyde, Carbamate, Carbonate, Urethane, Sulfonate, Sulfone, Sulfonamide, Nitro-Gruppen, Organosilan-Einheiten, Metallzentren, sauerstoff , stickstoff-, schwefel-, phosphorhaltige Heterocyclen.

13. Verfahren nach Anspruch 1-12, wobei die bi- oder polyfunktionellen Substraten eine oder mehrere primäre, sekundäre oder tertiäre basische Amin-Einheiten enthalten.

14. Verfahren nach Anspruch 1-13, wobei Mischungen der genannten Substrate umgesetzt werden, die als Gemisch oder sequentiell dem Reaktionsmedium zugeführt werden.

15. Verfahren nach Anspruch 1-14, wobei die genannten Substrate ganz oder teilweise in trägergebundener Form vorliegen.

16. Verfahren nach Anspruch 1-15, wobei die Produkte in einem integrierten Verfahren einer nachfolgenden Reaktion im komprimierten Kohlendioxid unterworfen werden.

17. Verfahren nach Anspruch 1-16, wobei das Reaktionsmedium zusätzlich ein oder mehrere Additiva enthält, die unabhängig gewählt werden aus: Wasser, Phosphorverbindungen, Amine, perfluorierte Verbindungen, Lewis-acide Verbindungen, Metallalkoxide, organische Lösungsmitteln (insbesondere Dichlormethan, Trichlormethan, Tetrachlormethan, 1,2-Dichlorethan, Trichlorethen, Benzol, Toluol, Xylol, Cumol, Hexan, Cyclohexan, Halogenbenzole, Tetrahydrofuran, *tert-*Butylmethylether, Diethylether, Dimethoxyethan, Dimethylformamid, Acetessigester, Aceton, Dimethylcarbonat, Alkohole).

18. Verfahren nach Anspruch 1 bis 17, wobel die Produkte durch Entspannen des komprimierten Kohlendioxids aus dem Reaktionsmedium abgetrennt werden.

19. Verfahren nach Anspruch 1-18, wobei die nach Abtrennen der chemischen Produkte verbleibenden metallhaltigen Rückstände erneut als Katalysatoren für Olefinmetathesen Anwendung findet.

20. Verfahren nach Anspruch 1-19, wobei das verwendete Kohlendioxid recycliert wird.

## Claims

1. A process for the preparation of cyclic products by the selective olefin metathesis of bi- or polyfunctional substrates in the presence of one or more homogeneous or heterogeneous metathesis catalysts in a reaction medium, **characterized in that** the substrates contain two or more functional groups in the form of substituted or unsubstituted alkene or alkyne units, and the reaction medium consists of compressed carbon dioxide, wherein the reaction temperature and the total pressure are balanced to provide a density of the reaction medium within a range of d = 0.4 - 1.5 g·cm⁻³, preferably within a range of d = 0.6 - 1.5 g·cm⁻³.

2. The process according to claim 1, wherein said products are carbo- or heterocyclic compounds having ring sizes of ≥ 5.

3. The process according to claim 2, wherein said products are carbo- or heterocyclic compounds having ring sizes of from 8 to 11.

4. The process according to claim 2, wherein said products are macrocyclic compounds having ring sizes of ≥ 12, especially pentadecanolide and homologues, dodecanedioic acid ethylene ester and homologues, cycloheptadec-9-ene-1-one and homologues, 3-methylcyclopentadecane-1-one and homologues, cyclopentadecanone and homologues, 3-methylcyclopentadec-5-ene-1-one and homologues, epothilone and homologues.

5. A process for the preparation of polymeric (including dimeric or oligomeric) products by the selective olefin metathesis of bi- or polyfunctional substrates in the presence of a homogeneous or heterogeneous metathesis catalyst in a reaction medium, **characterized in that** the substrates contain two or more functional groups in the form of substituted or unsubstituted alkene or alkyne units, and the reaction medium consists of compressed carbon dioxide, wherein the reaction temperature and the total pressure are balanced to provide a density of the reaction medium within a range of d = 0.2 - 1.3 g·cm⁻³, preferably within a range of d = 0.2 - 0.65 g·cm⁻³.

6. The process according to claims 1-5, wherein metathesis-active metal compounds which are not inactivated by compressed carbon dioxide are used as catalysts or catalyst precursors.

7. The process according to claim 6, wherein transition metal carbenes or transition metal compounds which form metal carbenes under the reaction conditions or transition metal salts in connection with an alkylating agent are used as catalysts or catalyst precursors.

8. The process according to claim 7, wherein compounds of general types I-IX 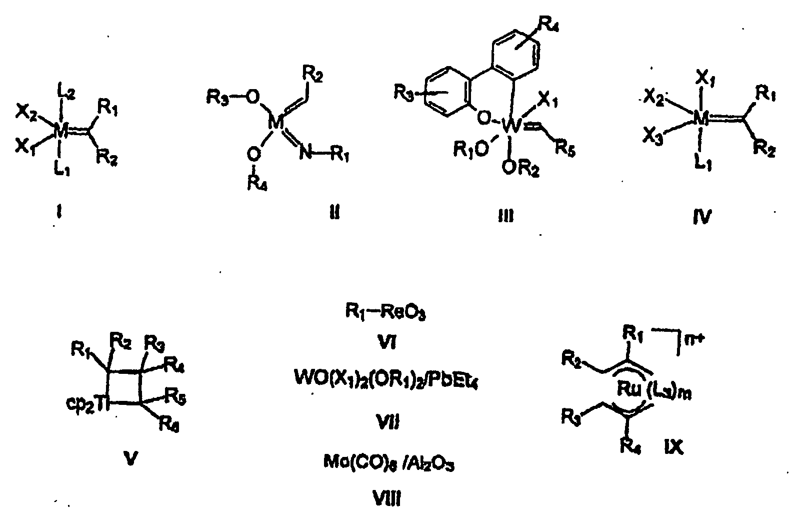 are used as catalysts or catalyst precursors, wherein:
R₁-R₆ are residues independently selected from hydrogen, C₂-C₂₀ alkenyl, C₂-C₂₀ alkynyl, C₁-C₂₀ alkyl, aryl, C₁-C₂₀ carboxylate, C₁-C₂₀ alkoxy, C₂-C₂₀ alkenyloxy, C₂-C₂₀ alkynyloxy, aryloxy, C₂-C₂₀ alkoxycarbonyl, C₁-C₂₀ alkylthio, C₁-C₂₀ alkylsulfonyl or C₁-C₂₀ alkylsulfinyl; each of which may optionally be substituted with C₁-C₁₂ alkyl, C₁-C₁₂ perfluoroalkyl, halogen, C₁-C₅ alkoxy or aryl; wherein residues R₁-R₆ may also be linked to one another in cyclic compounds;
X₁-X₃ are independently anionic ligands, especially selected from F⁻, Cl⁻, Br⁻, I⁻, CN⁻, SCN⁻, R₁O⁻, R₁R₂N⁻, (R₁-R₅)-allyl⁻, (R₁-R₅)-cyclopentadienyl⁻, wherein residues R₁-R₅ meet the definition mentioned above;
L₁-L₃ are independently neutral ligands, especially selected from CO, CO₂, R₁NCO, R₁R₂C=CR₃R₄, R₁C≡CR₂, R₁R₂C=NR₃, R₁C≡N, R₁OR₂, R₁SR₂, NR₁R₂R₃, PR₁R₂R₃, AsR₁R₂R₃, SbR₁R₂R₃; and
m and n are integers of between 0 and 6.

9. The process according to claim 8, wherein carbene complexes of general type I with L₁-L₂ = PR₁R₂R₃ are used as catalysts or catalyst precursors, wherein R₁-R₃ meet the definitions mentioned above, preferred residues R₁-R₃ being aryl or alkyl, especially secondary alkyl or cycloalkyl residues; wherein carbene complexes of general type II with R₁ = aryl and R₂-R₄ = C₁-C₂₀ alkyl, each of which may optionally be substituted with C₁-C₁₂ alkyl, C₁-C₁₂ perfluoroalkyl, aryl, halogen, are also preferred.

10. The process according to claims 1-9, wherein said bi- or polyfunctional substrates have a preorganized conformation or are completely flexible.

11. The process according to claims 1-10, wherein said bi- or polyfunctional substrates contain any number of further groups or heteroatoms which are compatible with the metathesis reaction, in addition to the functional groups participating in the reaction.

12. The process according to claim 11, wherein the groups or heteroatoms mentioned are independently selected from branched or unbranched alkyl residues, aromatic or non-aromatic carbocycles, carboxylic acids, esters, ethers, epoxides, silyl ethers, thioethers, thioacetals, anhydrides, imines, silyl enol ethers, ammonium salts, amides, nitriles, perfluoroalkyl groups, gem-dialkyl groups, alkynes, alkenes, halogens, alcohols, ketones, aldehydes, carbamates, carbonates, urethanes, sulfonates, sulfones, sulfonamides, nitro groups, organosilane units, metal centers, heterocycles containing oxygen, nitrogen, sulfur, phosphorus.

13. The process according to claims 1-12, wherein said bi- or polyfunctional substrates contain one or more primary, secondary or tertiary basic amine units.

14. The process according to claims 1-13, wherein mixtures of the mentioned substrates are reacted, being supplied to the reaction medium as a mixture or sequentially.

15. The process according to claims 1-14, wherein the mentioned substrates are wholly or partially in a supported form.

16. The process according to claims 1-15, wherein the products are subjected to a subsequent reaction in compressed carbon dioxide in an integrated process.

17. The process according to claims 1-16, wherein the reaction medium additionally contains one or more additives independently selected from water, phosphorus compounds, amines, perfluorinated compounds, Lewis-acidic compounds, metal alkoxides, organic solvents (especially dichloromethane, trichloromethane, tetrachloromethane, 1,2-dichloroethane, trichloroethene, benzene, toluene, xylene, cumene, hexane, cyclohexane, halobenzenes, tetrahydrofuran, tert-butyl methyl ether, diethyl ether, dimethoxyethane, dimethylformamide, acetacetic ester, acetone, dimethyl carbonate, alcohols).

18. The process according to claims 1-17, wherein the products are separated from the reaction medium by releasing the pressure from said compressed carbon dioxide.

19. The process according to claims 1-18, wherein the metal-containing residues remaining after the separation of the chemical products are again employed as catalysts for olefin metatheses.

20. The process according to claims 1-18, wherein the carbon dioxide is recycled after use.

## Revendications

1. Procédé de préparation de produits cycliques par métathèse sélective d'oléfines de substrats bi- ou polyfonctionnels en présence d'un ou de plusieurs catalyseurs de métathèse se présentant sous forme homogène ou hétérogène dans un milieu réactionnel, **caractérisé en ce que** les substrats contiennent deux ou plus de deux groupes fonctionnels sous la forme d'unités alcène ou alcyne substituées ou non substituées, et **en ce que** le milieu réactionnel se compose de dioxyde de carbone comprimé, la température de la réaction et la pression totale étant harmonisées entre elles de façon telle que la densité du milieu réactionnel se situe dans la gamme de *d =* 0,4 à 1,5 g.cm⁻³, de préférence dans la gamme de *d =* 0,6 à 1,5 g.cm⁻³.

2. Procédé selon la revendication 1, dans lequel les produits représentent des composés carbocycliques ou hétérocycliques présentant des tailles de cycle ≥ 5.

3. Procédé selon la revendication 2, dans lequel les produits représentent des composés carbocycliques ou hétérocycliques présentant des tailles de cycle de 8 à 11.

4. Procédé selon la revendication 2, dans lequel les produits représentent des composés macrocycliques présentant des tailles de cycle ≥ 12, en particulier des composés pentadécanolide et homologues, des composés éthylester de diacide de dodécane et homologues, des composés cycloheptadéc-9-én-1-ène et homologues, des composés 3-méthylcyclopentadécén-1-ène et homologues, des composés cyclopentadécénène et homologues, des composés 3-méthyl-cyclopentadéc-5-én-1-ène et homologues, et des composés épothilone et homologues.

5. Procédé de préparation de produits polymères (y compris des produits dimères ou oligomères) par métathèse sélective d'oléfine de substrats bi- ou polyfonctionnels en présence d'un catalyseur de métathèse se présentant sous forme homogène ou hétérogène dans un milieu réactionnel, **caractérisé en ce que** les substrats contiennent deux ou plus de deux groupes fonctionnels sous la forme d'unités alcène ou alcyne substituées ou non substituées, et **en ce que** le milieu réactionnel se compose de dioxyde de carbone comprimé, la température de la réaction et la pression totale étant harmonisées entre elles de façon telle que la densité du milieu réactionnel se situe dans la gamme de *d* = 0,2 à 1,3 g.cm⁻³, de préférence dans la gamme de *d* = 0,2 à 0,65 g.cm⁻³.

6. Procédé selon l'une des revendications 1 à 5, dans lequel on utilise en tant que catalyseurs ou précurseurs de catalyseurs, des composés métalliques actifs pour la métathèse et qui ne sont pas inactivés par du dioxyde de carbone comprimé.

7. Procédé selon la revendication 6, dans lequel on utilise en tant que catalyseurs ou précurseurs de catalyseurs, des carbènes de métaux de transition ou des composés de métaux de transition qui forment des carbènes métalliques dans les conditions de la réaction, ou des sels de métaux de transition en combinaison avec un agent d'alkylation.

8. Procédé selon la revendication 7, dans lequel on utilise en tant que catalyseurs ou précurseurs de catalyseurs, des composés correspondant aux types généraux I-IX : 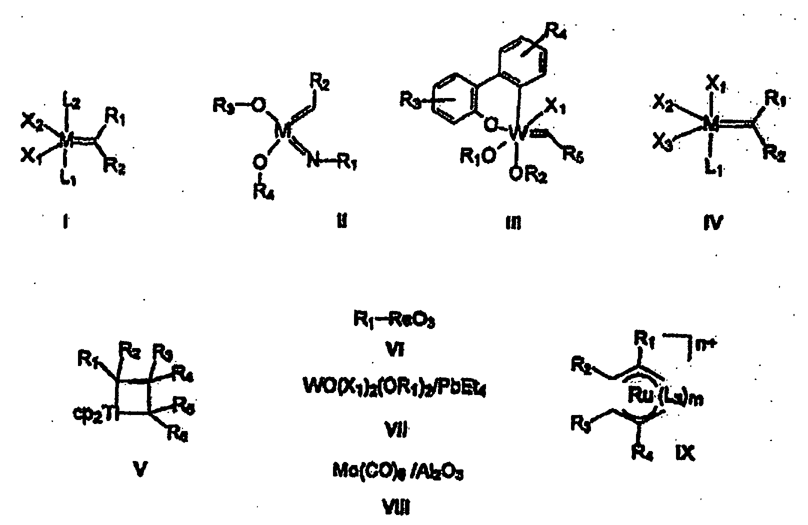
R₁ à R₆ sont des résidus pouvant être sélectionnés indépendamment l'un de l'autre parmi l'hydrogène, un alcényle en C₂ à C₂₀, un alcynyle en C₂ à C₂₀, un alkyle en C₁ à C₂₀, un aryle, un carboxylate en C₁ à C₂₀, un alcoxy en C₁ à C₂₀, un alcényloxy en C₂ à C₂₀, un alcynyloxy en C₂ à C₂₀, un aryloxy, un alcoxycarbonyle en C₂ à C₂₀, un alkylthio en C₁ à C₂₀, un alkylsulfonyle en C₁ à C₂₀ ou un alkylsulfinyle en C₁ à C₂₀; respectivement facultativement substitués par un alkyle en C₁ à C₁₂, un perfluoroalkyle en C₁ à C₁₂, un halogène, un alcoxy en C₁ à C₅, ou un aryle ; les résidus R₁ à R₆ peuvent se présenter liés ensemble dans des composés cycliques ;
X₁ à X₃ sont des ligands anioniques pouvant être sélectionnés indépendamment l'un de l'autre, en particulier pouvant être sélectionnés parmi F⁻, Cl⁻, Br⁻, I⁻, CN⁻, SCN⁻, R₁O⁻, R₁R₂N⁻, (R₁-R₅)-allyle⁻, (R₁-R₅)cyclopentadiényle⁻, les résidus R₁ à R₅ répondant à la définition déjà donnée ;
L₁ à L₃ sont des ligands neutres pouvant être sélectionnés indépendamment l'un de l'autre, en particulier pouvant être sélectionnés parmi CO,CO₂, R₁NCO, R₁R₂C=CR₃R₄, R₁C≡CR₂, R₁R₂C=NR₃, R₁C≡N, R₁OR₂, R₁SR₂, NR₁R₂R₃, PR₁R₂R₃, AsR₁R₂R₃, SbR₁R₂R₃ ;
m et n sont des nombres entiers entre 0 et 6.

9. Procédé selon la revendication 8, dans lequel on utilise en tant que catalyseurs ou précurseurs de catalyseurs, des complexes au carbène du type général I avec L₁-L₂ = PR₁R₂R₃, où R₁ à R₃ correspondent aux définitions précédemment données ; des résidus préférés R₁ à R₃ sont à cet égard un aryle ou un alkyle, en particulier des résidus alkyle secondaire ou cycloalkyle ; sont aussi préférés des complexes au carbène du type général II avec R₁ = aryle et R₂ à R₄ = alkyle en C₁ à C₂₀, respectivement facultativement substitués par un alkyle en C₁ à C₁₂, un perfluoroalkyle en C₁ à C₁₂, un aryle ou, halogène.

10. Procédé selon l'une des revendications 1 à 9, dans lequel les substrats bi- ou polyfonctionnels sont pré-organisés par conformation ou sont entièrement flexibles.

11. Procédé selon l'une des revendications 1 à 10, dans lequel les substrats bi- ou polyfonctionnels, outre les groupes fonctionnels prenant part à la réaction, contiennent un nombre quelconque d'autres groupes ou hétéroatomes compatibles avec la réaction de métathèse.

12. Procédé selon la revendication 11, dans lequel les groupes ou hétéroatomes nommés sont indépendamment sélectionnés parmi : des résidus alkyle ramifiés ou non ramifiés, des cycles carbocycliques aromatiques ou non aromatiques, des acides carboxyliques, des esters, des éthers, des époxydes, des silyléthers, des thioéthers, des thioacétals, des anhydrides, des imines, des silylénoléthers, des sels d'ammonium, des amides, des nitriles, des groupes perfluoroalkyle, des groupes gem-dialkyle, des alcynes, des alcènes, des halogènes, des alcools, des cétones, des aldéhydes, des carbamates, des carbonates, des uréthanes, des sulfonates, des sulfones, des sulfonamides, des groupes nitro, des motifs organosilane, des centres métalliques, des hétérocycles contenant de l'oxygène, de l'azote, du soufre ou du phosphore.

13. Procédé selon l'une des revendications 1 à 12, dans lequel les substrats bi- ou polyfonctionnels contiennent une ou plusieurs unités amine basique primaires, secondaires ou tertiaires.

14. Procédé selon l'une des revendications 1 à 13, dans lequel on fait réagir des mélanges des substrats nommés, qui sont amenés dans le milieu réactionnel sous forme de mélange ou en succession.

15. Procédé selon l'une des revendications 1 à 14, dans lequel les substrats nommés se présentent entièrement ou partiellement sous une forme liée au support.

16. Procédé selon l'une des revendications 1 à 15, dans lequel les produits, dans un procédé intégré, sont soumis à une réaction consécutive dans du dioxyde de carbone comprimé.

17. Procédé selon l'une des revendications 1 à 16, dans lequel le milieu réactionnel contient en outre un ou plusieurs additifs qui sont indépendamment sélectionnés parmi : de l'eau, des composés de phosphore, des amines, des composés perfluorés, des composés acides de Lewis, des alcoxydes métalliques et des solvants organiques (en particulier le dichlorométhane, le trichlorométhane, le tétrachlorométhane, le 1,2-dichloroéthane, le trichloroéthène, le benzène, le toluène, le xylène, le cumène, l'hexane, le cyclohexane, le benzène halogéné, le tétrahydrofuranne, le tert-butylméthyléther, le diéthyléther, le diméthoxyéthane, le diméthylformamide, l'ester d'acide acétique, l'acétone, le carbonate de diméthyle, des alcools).

18. Procédé selon l'une des revendications 1 à 17, dans lequel les produits sont séparés du milieu réactionnel par détente du dioxyde de carbone comprimé.

19. Procédé selon l'une des revendications 1 à 18, dans lequel les résidus contenant du métal qui subsistent après séparation des produits chimiques trouvent une nouvelle utilisation en tant que catalyseurs pour des métathèses d'oléfine.

20. Procédé selon l'une des revendications 1 à 19, dans lequel le dioxyde de carbone utilisé est recyclé.
